# EUROPEAN PATENT SPECIFICATION

(11) **EP 4 255 533 B1**
(45) Date of publication and mention of the grant of the patent: **30.10.2024**
(21) Application number: 21816091.9
(22) Date of filing: 30.11.2021
(51) Int. Cl.: A61M 5/31

(54) **INJECTION DEVICE FOR MEDICAL USE**
INJEKTIONSVORRICHTUNG FÜR MEDIZINISCHE ZWECKE
DISPOSITIF D'INJECTION À USAGE MÉDICAL

(30) Priority: 04.12.2020 IT 202000029984
(43) Date of publication of application: 11.10.2023
(73) Proprietor: Phitogen Beauty Labs SRL, 63074 San Benedetto Del Tronto (AP) (IT)
(72) Inventor: CIPOLLONI, Fabio, 63074 San Benedetto del Tronto (AP) (IT); GABRIELLI, Roberto, 63074 San Benedetto del Tronto (AP) (IT)
(74) Representative: Primiceri, Maria Vittoria
(86) International application number: PCT/EP2021/083487
(87) International publication number: WO 2022/117536

(56) References cited:
- EP-A1- 0 242 956
- WO-A1-2013/149032
- WO-A1-2019/099355
- DE-A1- 102005 005 468
- GB-A- 2 308 302
- US-B1- 10 842 940

## Description

The present invention relates to an injection device for medical use, and in particular for aesthetic medicine, for the injection of pasty products with high density and viscosity.

Syringes are a very important element in aesthetic medicine, as they are used by the physician to perform intracutaneous procedures. It must be considered that in aesthetic medicine it is often necessary to inject pasty products with high density and viscosity. As a result, the surgeon must exert a high pressure on the syringe in order to inject such products.

Two types of syringes are known in the field of aesthetic medicine: plastic and glass syringes.

Plastic syringes are provided with a plastic cylinder. Since plastic is a sufficiently elastic material, there is a very low risk of breaking the plastic cylinder during the injection.

Glass syringes are provided with a glass cylinder. Since glass is a rigid material, there is a high risk of breaking the glass cylinder during the injection.

However, glass has a characteristic that is fundamental in aesthetic medicine; in fact, glass is a material that maintains its sterility over time, whereas plastic is more subject to contamination.

WO2018/185406 discloses a syringe for medical aesthetics that comprises a flange applied on a plastic cylinder and a rod of a plunger provided with an oversized handgrip in such a way that the center of gravity of the syringe is located outside the cylinder. Such a syringe is impaired by some drawbacks due to the poor versatility of the handgrip which is not suitable for a traditional injection.

EP0242956A1 discloses a drug mixing apparatus comprising a syringe body, a flange, a rod and a holding disc fixedly mounted on a rear end of the rod. Such a holding disc cannot be removed from the rod and therefore is not interchangeable.

WO2019099355A1 discloses an injection device comprising a body, a rod with a plunger and two injection monitoring devices, namely a first monitoring device disposed on a rear end of the rod, and a second monitoring device disposed on the syringe body and acting as flange. The first monitoring device is removably coupled with the rear end of the rod in axial fit-in coupling mode; otherwise said, in order to assemble the monitoring device, the user must press the monitoring device axially on the rear end of the rod, whereas in order to disassemble the monitoring device, the user must pull the monitoring device axially from the rear end of the rod. Such injection device is impaired by the fact that during the extraction of a fluid, the user must pull the rod backwards, exerting a force on the first monitoring device in opposite direction to the assembling direction, with the risk of disassembling the first monitoring device from the rod. Pasty fluids are used in aesthetic surgery and a high force is required during the injection and the extraction. Therefore, such an injection device cannot be used in aesthetic surgery.

GB2308302A discloses an injection device provided with a syringe body, a rod and a handgrip removably mounted on a rear end of the rod in axial fit-in coupling mode. Just like the injection device of document WO2019099355A1, also the injection device of GB2308302A is impaired by the fact that during the extraction, the handgrip can be detached from the rod because of the axial coupling mode.

US10842940 discloses a syringe, comprising a syringe barrel, a syringe plunger and an adhesive ring connected to a rear end of the plunger that allows for inserting the thumb for making it easier to pull the syringe plunger with only one hand. Evidently, the adhesive coupling is weak and also in this case, during the extraction, there is a risk of uncoupling the adhesive ring from the rear end of the plunger.

WO2013149032A1 discloses a delivery device comprising a barrel and a plunger having a rear enlarged portion. The enlarged portion cannot be disassembled from the plunger.

DE 10 2005 005468 discloses an improved safety syringe comprising a syringe cylinder and two-piece finger support which exhibits supporting and bolting device elements.

It is the purpose of the present invention to eliminate the drawbacks of the prior art by providing an injection device that is reliable, practical, versatile, and quick and easy to assemble.

Another purpose of the present invention is to provide such an injection device that is particularly suitable for being used in aesthetic surgery in which pasty fluids are aspirated and injected.

These purposes are achieved in accordance with the invention with the features of the independent claim 1.

Advantageous embodiments of the invention appear from the dependent claims.

Further features of the invention will appear clearer from the following detailed description, which refers to a merely illustrative and therefore non-limiting embodiment thereof, illustrated in the appended drawings, wherein:
Fig. 1 is an exploded perspective view of the various components of the injection device according to the invention,
Fig. 2 is a perspective view of a first removable handgrip of the injection device of Fig. 1;
Fig. 3 is an axial sectional view of the first removable handgrip of Fig. 2;
Fig. 4 is a bottom view of the first removable handgrip of Fig. 2;
Fig. 5 is a perspective view of a second removable handgrip of the injection device of Fig. 1;
Fig. 6 is a bottom view of the second removable handgrip of Fig. 5;
Fig. 7 is a perspective view of a third removable handgrip and of the head of a stem of the injection device uncoupled from each other;
Fig. 7A is a bottom view of the head of the stem of Fig. 7;
Fig. 7B is a bottom view of the third handgrip of Fig. 7;
Fig. 8 is a perspective view of the third handgrip and of the head of Fig. 7 coupled with each other;
Fig. 9 is a perspective view of a flange of the injection device of Fig. 1;
Fig. 10 is an axial cross-sectional view of the flange of Fig. 9;
Fig. 11 is a bottom view of the flange of Fig. 10;
Fig. 12 is a perspective view, illustrating the injection device of Fig. 1 in assembled condition, in a first configuration, without removable flange;
Fig. 13 is a perspective view illustrating the use of the device of Fig. 12 in the first configuration;
Fig. 14 is a perspective view illustrating the injection device of Fig. 1 in assembled condition, in a second configuration, with the first removable handgrip;
Fig. 15 is a perspective view illustrating the use of the device of Fig. 14 in the second configuration;
Fig. 16 is a perspective view illustrating the injection device of Fig. 1 in assembled condition, in a third configuration, with the second removable handgrip; and
Fig. 17 is a perspective view illustrating the use of the device of Fig. 16 in the third configuration;
Fig. 18 is a perspective view illustrating the injection device of Fig. 1 in assembled condition, in a fourth configuration, with the third removable handgrip;
Figs. 19 and 20 are perspective views illustrating the use of the device of Fig. 18 in the fourth embodiment.

Referring to the Figures, the injection device according to the invention is disclosed, which is generally indicated with reference numeral 100

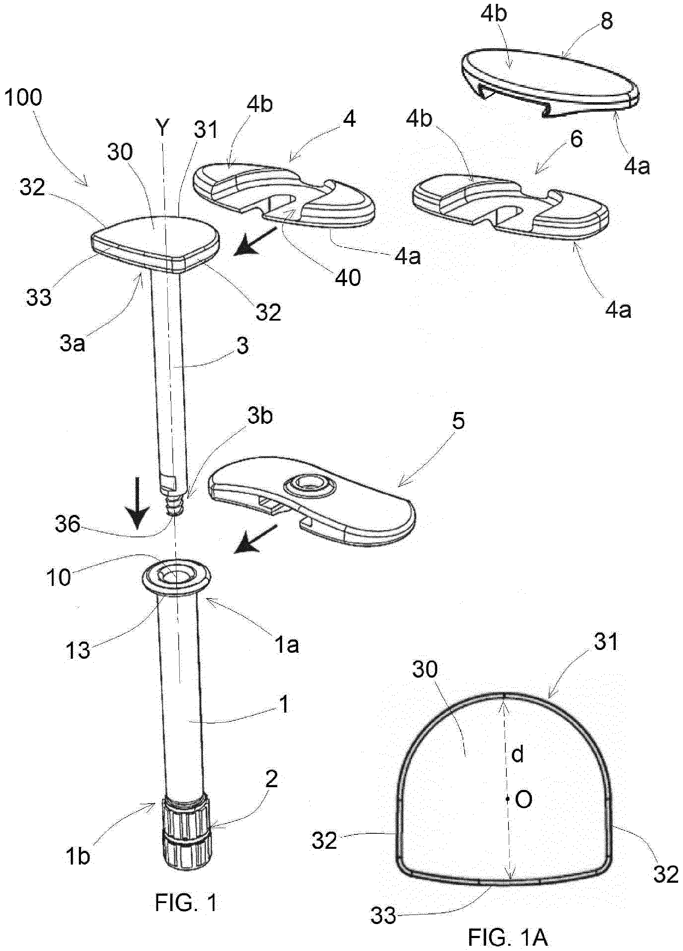

With reference to Fig. 1, the injection device (100) according to the invention comprises
- a cylinder (1) with an internal chamber (10),
- a stem (3) suitable for sliding inside the chamber (10) of the cylinder;
- a flange (5) suitable for being removably fixed to the cylinder (1), and
- at least one handgrip (4; 6), of interchangeable type, suitable for being removably fixed to the stem (3) in fit-in coupling mode; said at least one handgrip (4; 6; 8) comprises a lower side (4a) suitable for facing the cylinder (1) and an upper side (4b) opposite to said lower side (4a).

In the following description, the terms "proximal" and "distal" refer to the distance of an element of the injection device with respect to a user that uses the injection device.

The cylinder (1) is advantageously made of glass. The cylinder (1) has a proximal end (1a) and a distal end (1b).

An injection needle (not shown in the figures) is disposed at the distal end (1b) of the cylinder. The needle has a channel in communication with the chamber (10) of the cylinder for injecting the material contained in the chamber of the cylinder.

The needle may be connected to the distal end (1b) of the cylinder by means of an attachment (2), such as a Luer attachment, which is of known type and therefore is not explained in detail.

A collar (13) protrudes radially outwards from the proximal end (1a) of the cylinder. The collar (13) has an annular shape.

The stem (3) has a proximal end (3a) and a distal end (3b).

An attachment (36) is disposed at the distal end (3b) of the stem for being attached to a plunger (not shown in the figures) that slides tightly inside the chamber (10) of the cylinder. The plunger may be made of a soft material, such as rubber, is of known type and therefore is not shown.

A head (30) is disposed at the proximal end (3a) of the stem. The head (30) is shaped like a plate arranged orthogonally with respect to an axis of the stem.

Also with reference to Fig. 1A, the head (30) has a substantially semi-elliptical shape, namely the shape of a fingertip of a thumb of a hand. The head (30) has an upper side having an area that is substantially equal to the area of a fingertip of a thumb of a hand.

The head (30) has a rear edge (31), two lateral edges (32) and a front edge (33). The rear edge (31) is curved and shaped like an arc of a circle with a center (O) disposed in correspondence of the center of gravity of the head (30) and of the axis of the stem (3). The lateral edges (32) are straight. The front edge (33) is substantially straight and is exactly shaped like a central section of an ellipse.

By way of example, the front edge (33) is 21 - 25 mm long, preferably 23.12 mm long. The rear edge (31) is shaped like a semicircle with radius 10-13 mm, preferably 11.46 mm. Between the front edge (33) and the rear edge (31) there is a maximum distance (d) which is 19 - 23 mm long, preferably 21 mm.

The injection device (100) comprises a first handgrip (4) shaped like a plate with a recessed housing (40) suitable for accommodating the head (30) of the stem.

The recessed housing (40) is formed on the upper side (4b) of the first handgrip.

The recessed housing (40) has an opening (400) that is formed on a perimeter edge of the first handgrip (4) in such a way that the first handgrip (4) is coupled with the head (30) of the stem (3) in a radial direction to the axis of the stem (3).

Also the uncoupling of the first handgrip relative to the head of the stem occurs in a radial direction with respect to the axis of the stem. Such a solution avoids the accidental uncoupling of the first handgrip from the head of the stem when the stem is pulled, especially when pasty fluids are used and a high pulling force is required.

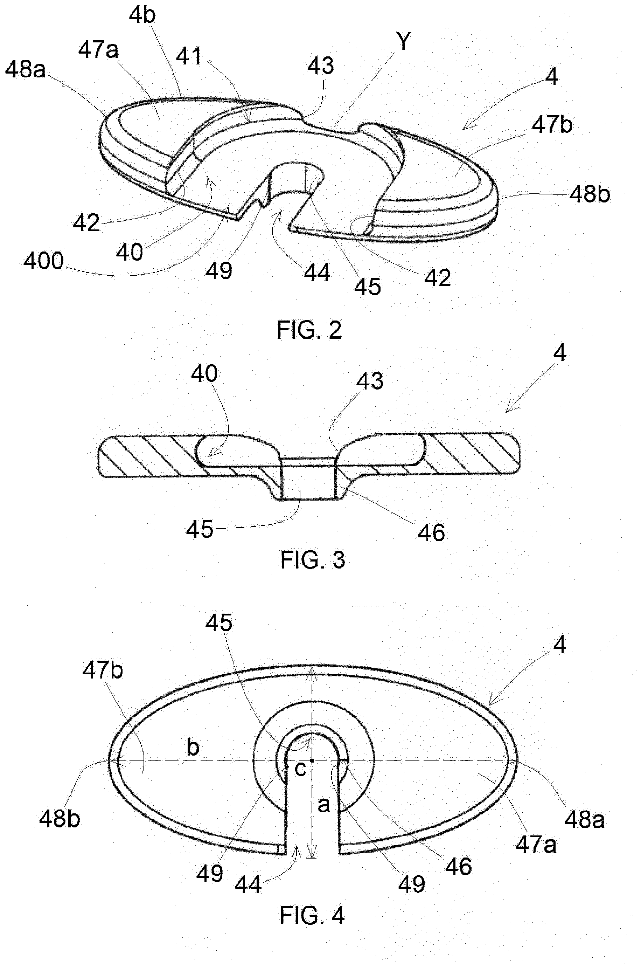

With reference to Figs. 2, 3 and 4, the recessed housing (40) of the first handgrip (4) has the same shape as the head (30).

The recessed housing (40) of the first handgrip (4) comprises two straight lateral edges (42) that are joined to a curved rear edge (41). The depth of the recessed housing (40) is equal to the thickness of the head (30).

An opening (43) is provided in the center of the curved rear edge (41). In this way, the curved profile of the front edge (33) of the head (30) can protrude from the opening (43), once the head (30) is inserted into the recessed housing (40) of the handgrip. Thus, the curved profile of the front edge (33) of the head (30) is perfectly aligned with the curved profile of the rear edge (41) of the handgrip (4). The opening (43) of the handgrip is also used to remove superfluous material from the handgrip (4).

A U-shaped slot (44), which is open at the front, is provided in the center of the recessed housing (40) The slot (44) ends in a hole (45) provided in the center of the handgrip. In the slot (44) there are two retention teeth (49) that protrude into the slot, in diametrically opposite positions, at the entrance to the hole (45). The retention teeth (49) are elastically deformable.

The first handgrip (4) is attached to the stem (3) in fit-in coupling mode by deforming the retention teeth (49). The stem (3) is inserted into the slot (44) and forces the retention teeth (49). The lateral edges (32) of the head slide in the lateral edges (42) of the housing of the first handgrip until the stem (3) passes beyond the retention teeth and enters the hole (45). The rear edge (31) of the head abuts against the rear edge (41) of the housing of the first handgrip, locking the head (30) into the recessed housing (40) of the first handgrip. The retention teeth (49) hold the stem (3), preventing it from being released from the hole (45).

Advantageously, the lateral edges (42) of the recessed housing of the handgrip have a concave C-shaped cross-section, so as to retain the handgrip relative to the head and prevent the handgrip from being axially released downwards with respect to the head.

Referring to Fig. 4, a collar (46), which is shaped like an open ring, protrudes inferiorly from the first handgrip (4) around the hole (45), acting as a reinforcement to retain the stem (3).

Two lateral elements (47a, 47b) protrude in opposite directions from the housing (40) of the first handgrip.

Referring to Fig. 4, the first handgrip (4) has an elliptical shape, with a minor axis (a) and a major axis (b) crossing at a point (C) corresponding with the center of the hole (45). The minor axis (a) corresponds with the axis of the slot (44). The major axis (b) extends into the two lateral elements (47a, 47b).

The minor axis (a) of the handgrip has a length equal to the maximum distance (d) between the front edge (33) and the rear edge (31) of the head, i.e. the minor axis (a) has a length of 16-24 mm. The major axis (b) is approximately twice as long as the minor axis (a), i.e., the major axis has a length of 32 - 44 mm.

The two lateral elements (47a, 47b) of the first handgrip (4) are shaped like portions of an ellipse, with curved edges (48a, 48b). Such ergonomic configuration of the first handgrip, perfectly fits to a portion of the palm of a hand in correspondence of the thumb.

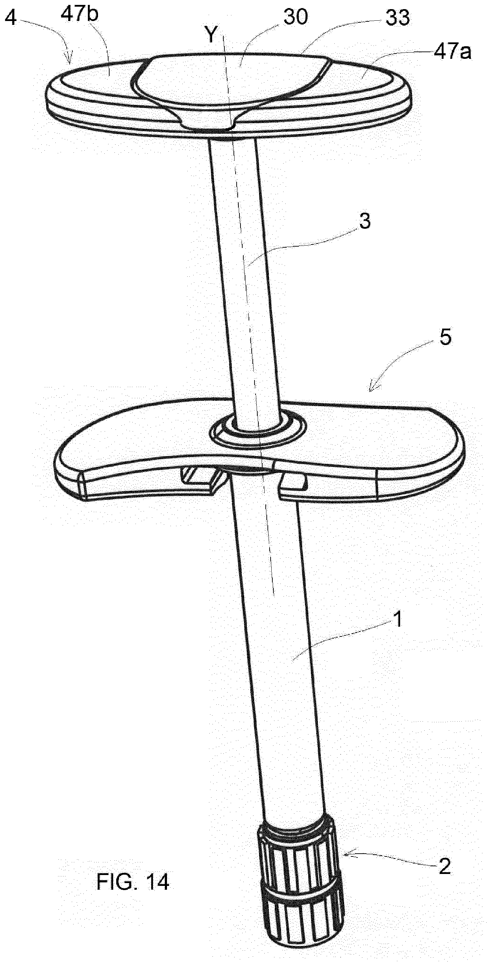

With reference to Fig. 14, it should be noted that when the first handgrip (4) is attached to the head (30) of the stem, the front edge (33) of the head is flush with the edges of the two lateral elements (47a, 47b). Since the front edge (33) of the head is shaped like a section of an ellipse, no corner is formed between the lateral elements (47a, 47b) and the head (30) of the stem.

The rear edge (31) of the head is flush with the opening (43) of the handgrip, preventing the formation of corners.

Furthermore, since the depth of the recessed housing (40) is equal to the thickness of the head, the upper surface of the head (30) is flush with the upper surface of the lateral elements (47a, 47b).

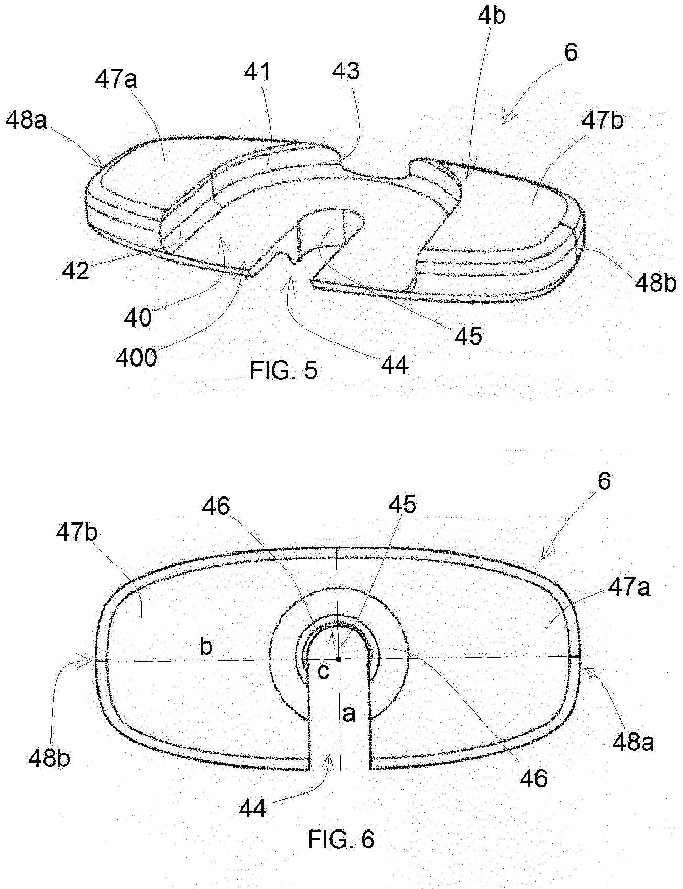

With reference to Figs. 5 and 6, the second handgrip (6) is substantially the same as the first handgrip (4); therefore, equal or corresponding elements are shown with the same reference numbers, omitting their detailed description.

The second handgrip (6) differs from the first handgrip (4) in that it has a rectangular or substantially elliptical shape with the lateral elements (47a, 48b) having straight edges (48a, 48b), rather than rounded edges. It should be noted that in this way the second handgrip (6) is substantially shaped like a thumb of a hand.

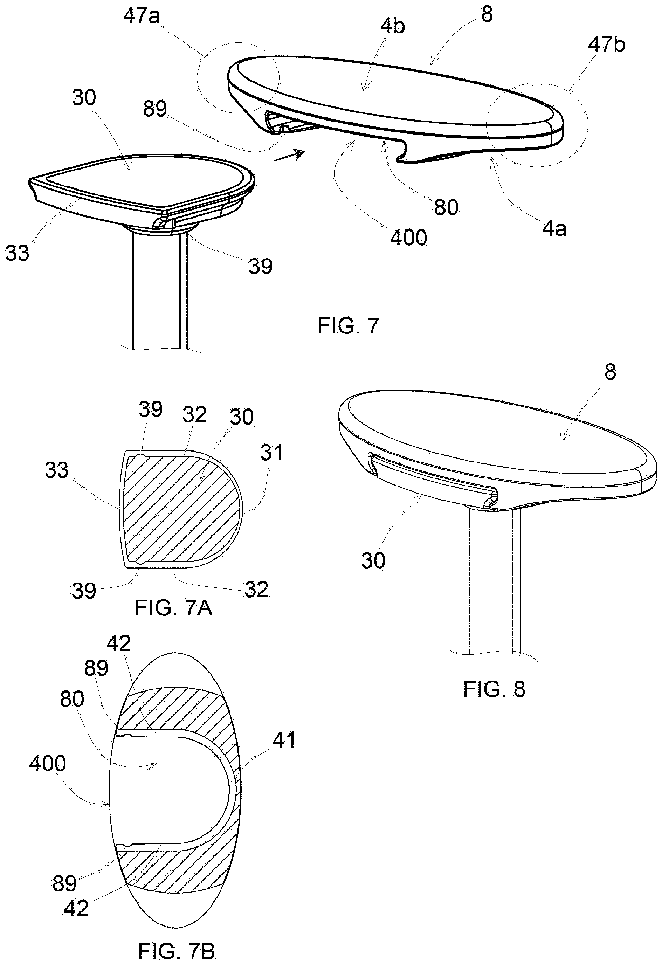

With reference to Figs. 7, 7B and 8, the third handgrip (8) differs from the first handgrip (4) and from the second handgrip (6) in that the recessed housing (80) is formed on the lower side (4a) of the third handgrip (8) rather than on the upper face, in such a way that the third handgrip (8) completely covers the head (30) of the stem on top.

However, the recessed housing (80) comprises the two straight lateral edges (42) that are joined with a curved rear edge (41), wherein said lateral edges (42) have a concave C-shaped section, and said recessed housing (40) has a depth equal to the thickness of the head (30).

With reference to Figs. 7A and 7B, in such a case the third handgrip (8) comprises two opposite notches (89) formed on the lateral edges (42) of the recessed housing (80). The head (30) has teeth (39) that protrude externally from the straight lateral edges (32). The teeth (39) of the head engage the notches (89) of the third handgrip.

Referring to Figs. 7, 7B and 8, the third handgrip (8) has an elliptical shape, and said lateral elements (47a, 47b) are provided with rounded edges (48a, 48b). The shape of the third handgrip (8) is substantially identical to the shape of the first handgrip (4).

Nevertheless, the third handgrip (8) may also have a shape similar to that one of the second handgrip (6), that is to say a substantially rectangular shape with the lateral edges provided with straight edges.

Compared to the first handgrip (4) and to the second handgrip (6), the third handgrip (8) provides a more stable and more secure coupling, completely eliminating the possibility that the third handgrip (8) may be disengaged from the head (30) of the stem.

The first handgrip, the second handgrip and the third handgrip (4, 6, 8) are advantageously made of two materials: a harder material, such as polycarbonate, and a softer material, such as a thermoplastic elastomer. The thermoplastic elastomer may be in the form of strips on the external surface of the first handgrip and of the handgrip. Such a result can be achieved with molding techniques of two materials according to the prior art.

The provision of thermoplastic elastomer strips ensures a higher friction and improves the grip with the physician's hands or gloves.

In particular, thermoplastic elastomer strips are provided in the lower surface of the first handgrip, of the second handgrip, and of the third handgrip (4, 6, 8) to improve the grip with the physician's fingers during the extraction of the injection device (100).

The stem (3) is made of polycarbonate, whereas the head (30) can be made of two materials, like the handgrips (4, 6, 8).

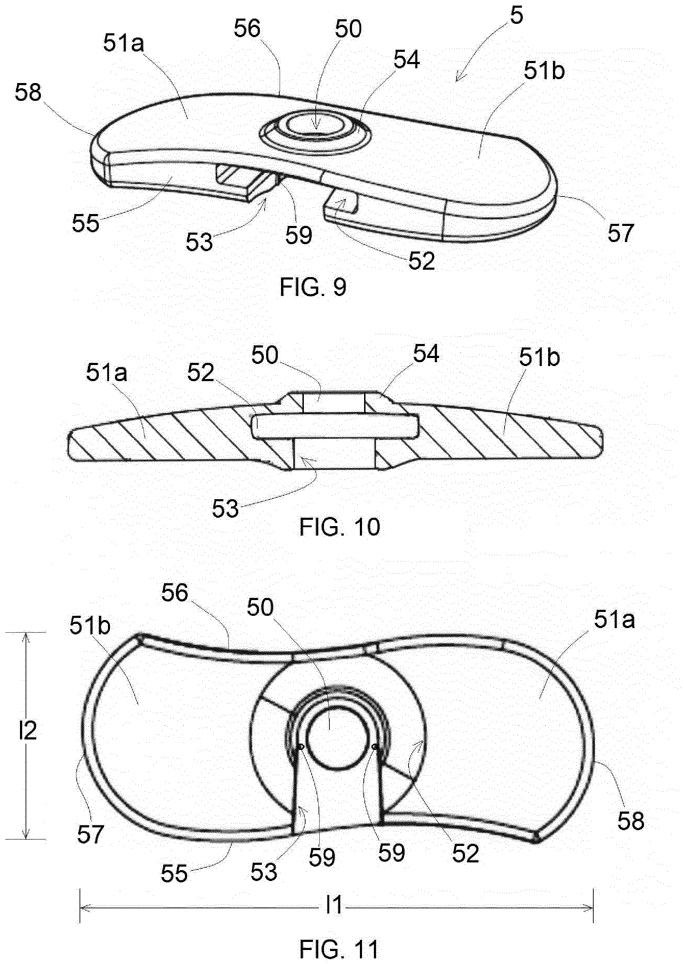

With reference to Figs. 9, 10 and 11, the flange (5) has a hole (50) arranged in a central position for the insertion of the stem (3). Two lateral elements (51a, 51b) protrude in opposite directions from the hole (50). The lateral elements (51a, 51b) have a length greater than the width of the index and middle fingers to improve the physician's grip.

The flange (5) has an anterior edge (55) and a posterior edge (56) in the shape of a bend. The anterior and posterior edges (55, 56) are joined with lateral edges (57, 58) shaped like an arc of a circle.

The flange (5) has a seat (52) in communication with the hole (50) and open at the anterior edge (55). The seat (52) has a section shaped like an inverted "C" and is suitable for accommodating and holding the collar (13) of the cylinder.

A "U" shaped slot (53) is formed in a lower wall of the flange in such a way to communicate with the seat (52). The slot (53) allows for the insertion and passage of the cylinder (1). Two retention teeth (59) protrude into the slot (53) in opposite directions. The retention teeth (59) are deformed as the cylinder (1) passes through and retain the cylinder (1) under the collar (13) in fit-in coupling mode.

A collar (54) protrudes superiorly from the flange around the bore (50) to guide the sliding of the stem (3). The collar (54) prevents a clearance of the stem (3) during the injection that can cause the unbalancing of the plunger or an excessive effort of the physician in administering thicker products, or the breakage of the cylinder (1) if it is made of glass. The provision of the collar (54) in the flange allows for a guided uniform injection, avoiding breaking the cylinder if it is made of glass.

The flange (5) has a length (11) of 45-55 mm and a width (I2) of 16-24 mm (at the center of the hole (50)).

The flange (5) can also be made of two materials, like the handgrips (4, 6, 8).

The use of the injection device (100) is described below.

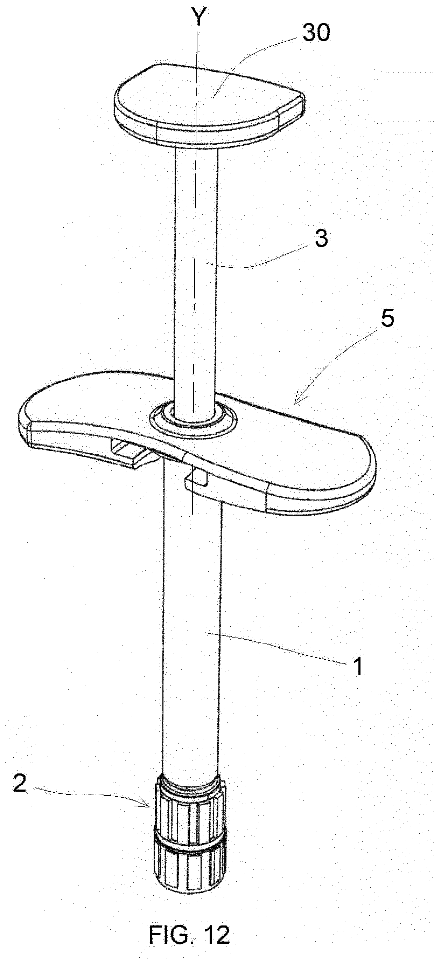

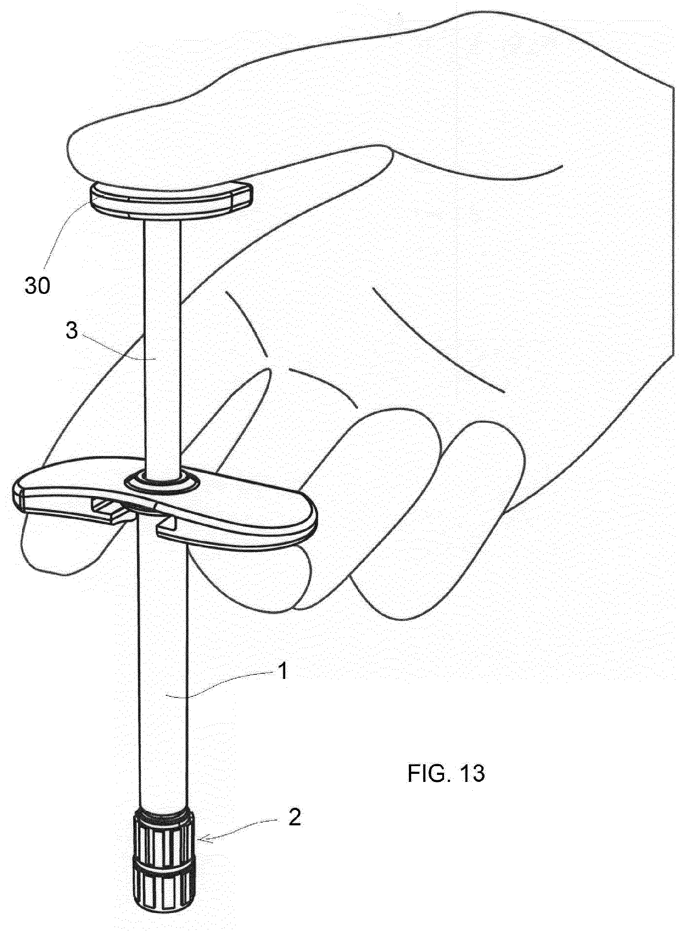

Referring to Figs. 12 and 13, in the case where products with a low density and a low viscosity are to be injected, the administration of which requires no effort in the injection, and in the case where the injection is to be performed in parts of the body where a greater precision is required, the physician will use the injection device (100) without any removable handgrip.

In such a case, the reduced surface of the head (30) of the stem allows the physician to inject with his thumb. In fact, the shape of the head (30) adapts perfectly to the fingertip of the thumb. With such a first configuration of the injection device, the physician will use a lightweight handgrip that is ideal for injecting low density products with high precision.

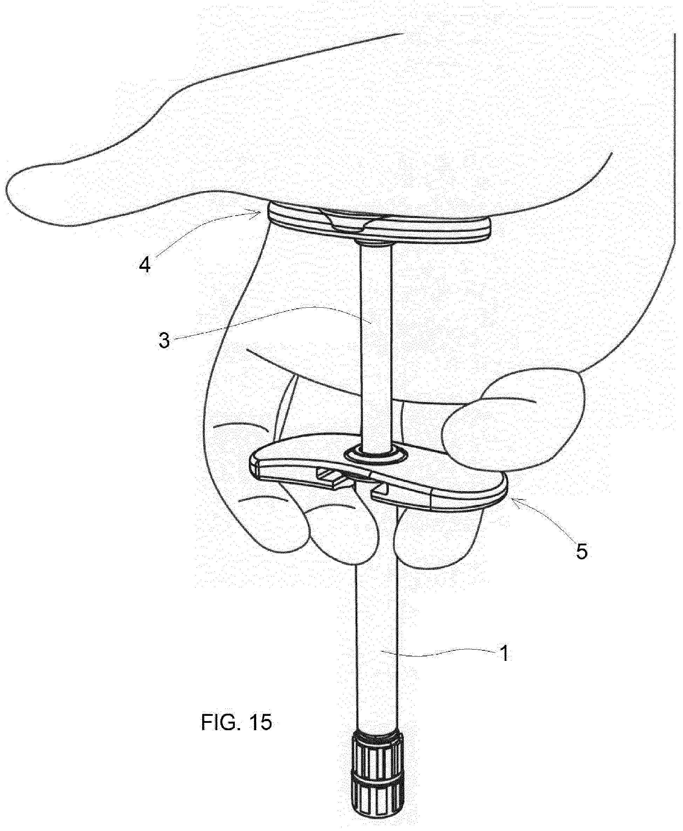

With reference to Figs. 14 and 15, in the case where products with a high density and a high viscosity are to be injected, which require a high effort from the physician, the physician will use the injection device (100) with the first handgrip (4) of elliptical shape that allows the physician to inject with the palm of the hand. Such an injection technique allows for exerting a greater force on the first handgrip (4) for the administration of products with a high density and a high viscosity. The special ergonomic shape (elliptical shape) of the first handgrip, which adapts to the conformation of the palm of the hand, ensures a firm grip and facilitates a controlled homogeneous injection of the product, reducing the physician's effort.

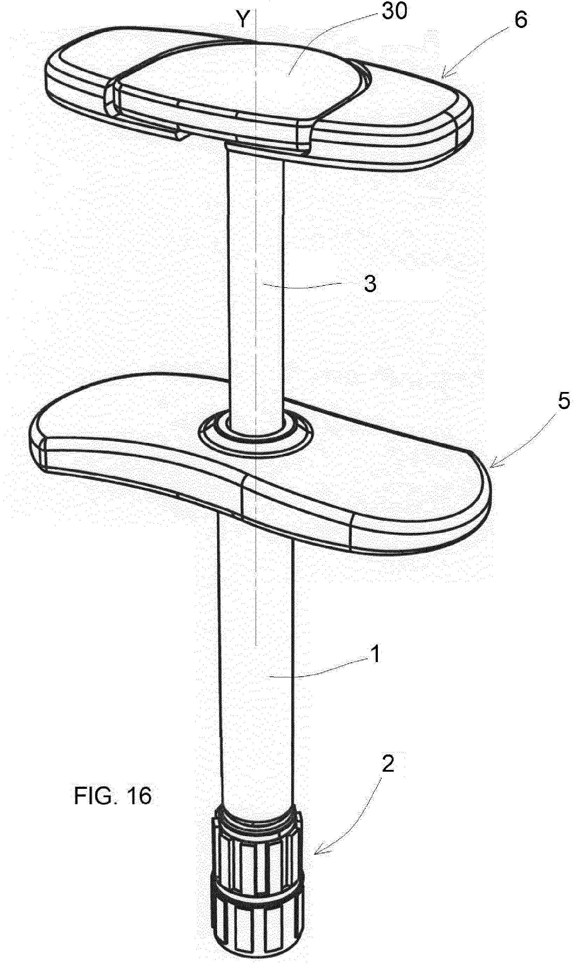

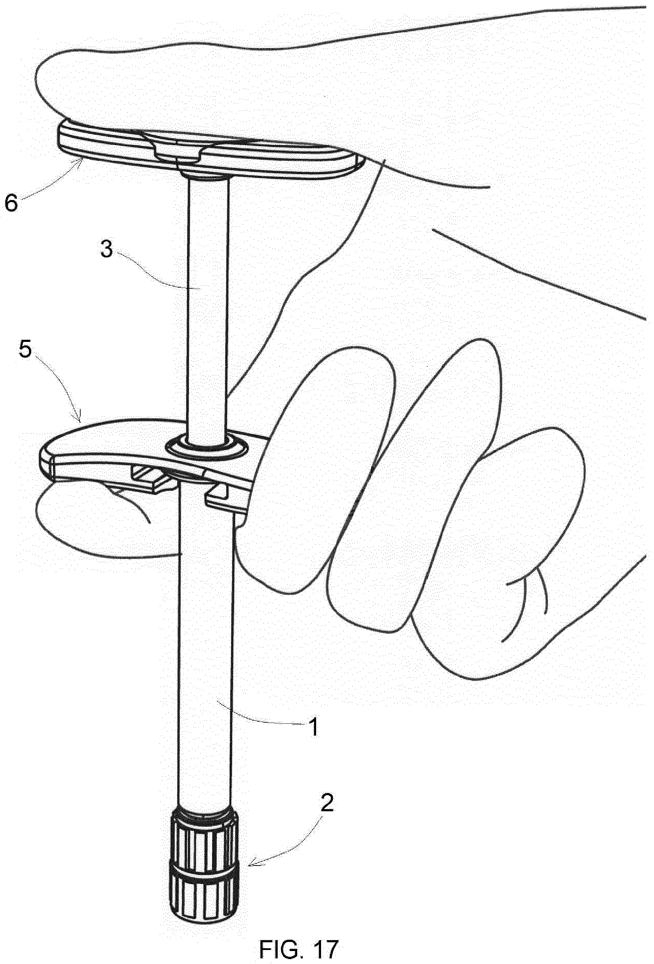

With reference to Figs. 16 and 17, in the case where products with a medium density and a medium viscosity are to be injected, which require a medium effort by the physician, the physician will use the injection device (100) with the second handgrip (6) with a rectangular or elliptical shape (6) with straight edges that reproduces the shape of the thumb. Such a second handgrip (6) allows the physician to inject with the entire surface of the thumb.

Such an injection technique ensures a firmer grip of the thumb on the second handgrip (6) with a uniform distribution of all pressure forces over the entire surface of the second handgrip (6) and is thus ideal for the administration of products with a medium density that require a medium effort for the injection.

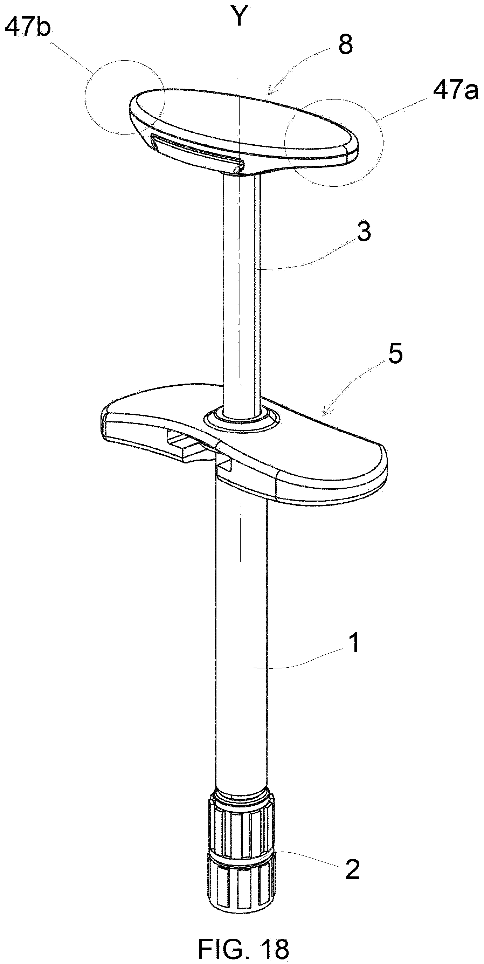

With reference to Figs. 18, 19 and 20, the injection device with the third handgrip (8) is illustrated, alongside with two of its uses.

The third handgrip (8) is more versatile and safer than the first handgrip and the second handgrip (4, 6).

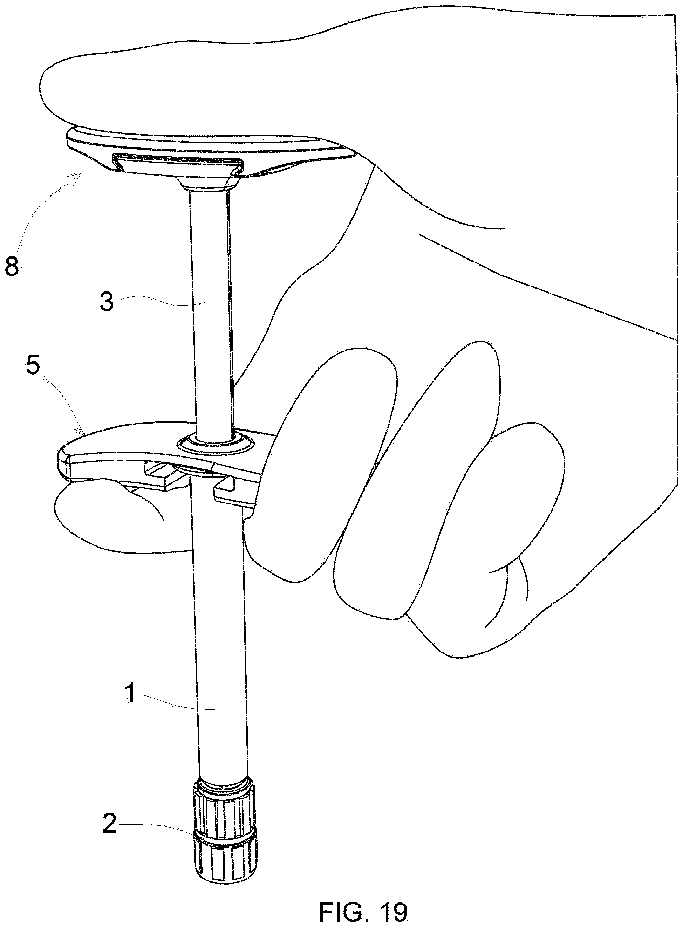

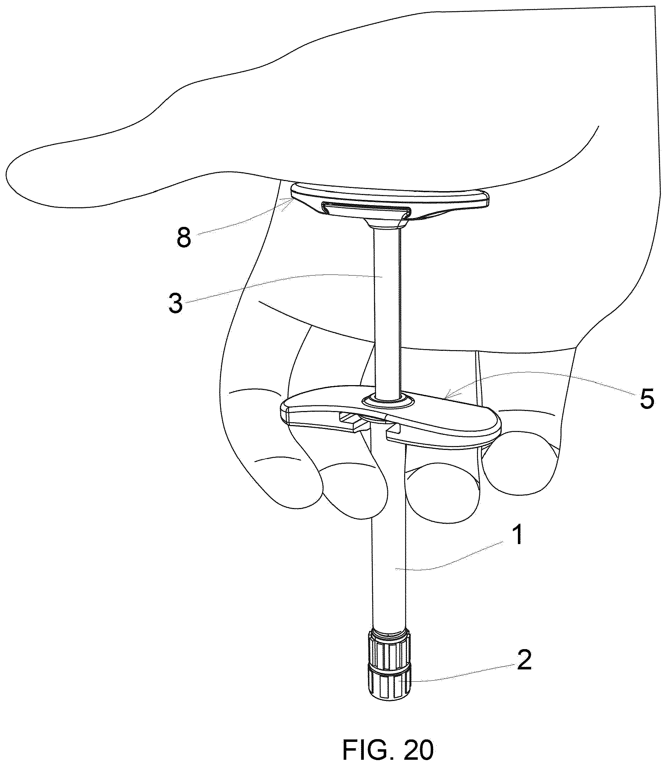

In fact, the third handgrip (8) can be used to inject products with a medium density, using the entire palm of the hand (see Fig. 20) as well as products with a high density, using the thumb of the hand (see Fig. 19).

Evidently, the fact that the handgrips are coupled with the head (30) of the stem (3) in a radial direction relative to the axis of the stem (3) drastically reduces the risks that the handgrip is uncoupled from the head when it is pulled to extract the fluid.

Equivalent variations and modifications may be made to the present embodiments of the invention, within the scope of a person skilled in the art, and in any case falling within the scope of the invention as expressed by the appended claims.

## Claims

1. An injection device (100) comprises:
- a cylinder (1) with an internal chamber (10),
- a stem (3) suitable for sliding inside the chamber (10) of the cylinder; said stem having a proximal end (3a) with a head (30) shaped like a plate that is orthogonal to an axis (Y) of the stem;
- a flange (5) suitable for being removably fixed to the cylinder (1),
- at least one handgrip (4; 6), of interchangeable type, suitable for being removably fixed to the stem (3), in fit-in coupling mode; said handgrip (4, 6, 8) comprises a lower side (4a) suitable for facing the cylinder (1) and an upper side (4b) opposite to said lower side (4a); said at least one handgrip (4, 6, 8) comprises a recessed housing suitable for accommodating said head (30) of the stem;
**characterized in that**
said recessed housing (40, 80) has an opening (400) that is obtained on a perimeter edge of said handgrip (4; 6; 8) in such a way that said handgrip (4; 6; 8) is coupled and uncoupled with said head (30) of the stem (3) in a radial direction with respect to the axis (Y) of the stem (3).

2. The injection device (100) according to claim 1, wherein said at least one handgrip (4, 6, 8) comprises two lateral elements (47a, 47b) that protrude in opposite directions from said recessed housing (40, 80).

3. The injection device (100) according to any one of the preceding claims, wherein said head (30) of the stem has a substantially semi-elliptical shape, like a fingertip of a thumb; said head (30) comprising:
- a curved rear edge (31) shaped like an arc of circle with a center (O) disposed in correspondence of the axis of the stem (3),
- two straight lateral edges (32), and
- a front edge (33) that is substantially straight or shaped like a central portion of an ellipse.

4. The injection device (100) according to claim 3, wherein said recessed housing (40, 80) comprises two straight lateral edges (42) that are joined to a curved rear edge (41), wherein said lateral edges (43) have a concave C-shaped section, and the depth of said recessed housing (40) is equal to the thickness of the head (30).

5. The injection device (100) according to any one of the preceding claims, wherein said recessed housing (40, 80) is obtained on the upper side (4b) of said at least one handgrip (4, 6); said at least one handgrip (4, 6) comprising a slot (44) that is provided in central position in said recessed housing; said slot (44) being open in the front and in the bottom and ending in a hole (45) to accommodate said stem (30).

6. The injection device (100) according to claim 5, wherein said at least one handgrip (4; 6) comprises retention teeth (49) that protrude inside said slot (44) and are elastically deformed in order to retain said stem (3).

7. The injection device (100) according to claim 5 or 6, wherein said at least one handgrip (4; 6) comprises a collar (46) shaped like an open ring that protrudes in lower position around said hole (45).

8. The injection device (100) according to any one of claims 5 to 7, wherein said at least one handgrip comprises a first handgrip (4) with an elliptical shape, said lateral elements (47a, 47b) being provided with rounded edges (48a, 48b).

9. The injection device (100) according to any one of claims 5 to 7, wherein said at least one handgrip comprises a second handgrip (6) having a rectangular or a substantially elliptical shape, said lateral elements (47a, 47b) being provided with straight edges (48a, 48b).

10. The injection device (100) according to any one of claims 1 to 4, wherein said recessed housing (80) is obtained on the lower side (4a) of the handgrip (8) in such a way that said handgrip (8) completely covers the head (30) on top.

11. The injection device (100) according to claim 10, wherein said handgrip (8) comprises opposite notches (89) obtained on the lateral edges (42) of the recessed housing (80); said head (30) comprising teeth (39) that protrude externally from the straight lateral edges (32) and are engaged in the notches (89) of the handgrip (8).

12. The injection device (100) according to any one of claims 10 to 11, wherein said at least one handgrip comprises a third handgrip (8) with an elliptical shape, said lateral elements (47a, 47b) being provided with rounded edges (48a, 48b)..

13. The injection device (100) according to any one of the preceding claims, wherein said flange (5) comprises:
- a hole (50) disposed in central position, suitable for being penetrated by the stem (3);
- two lateral elements (51a, 51b) that protrude in opposite directions from the hole (50),
- a seat (52) in communication with the hole (50) and open in the front to accommodate and retain a collar (13) of the cylinder (1),
- a U-shaped slot (53) obtained in a lower wall of the flange, in such a way to communicate with the seat (52) for the insertion and the passage of the cylinder (1),
- retention teeth (59) that protrude in the slot (53) in opposite directions to retain the cylinder (1), and
- a collar (53) shaped like a closed ring that protrudes in upper position from the flange around the hole (50) to guide the sliding movement of the stem (3).

14. The injection device (100) according to any one of the preceding claims, wherein said cylinder (10) is made of glass.

## Patentansprüche

1. Injektionsvorrichtung (100), umfassend:
- einen Zylinder (1) mit einer Innenkammer (10),
- eine Stange (3), die in der Kammer (10) des Zylinders gleiten kann; wobei die Stange ein proximales Ende (3a) mit einem plattenförmigen Kopf (30) aufweist, der orthogonal zu einer Achse (Y) der Stange ist;
- einen Flansch (5), der abnehmbar am Zylinder (1) befestigt werden kann,
- mindestens einen Handgriff (4; 6) von austauschbarem Typ, der im formschlüssigen Kopplungsmodus abnehmbar an der Stange (3) befestigt werden kann; wobei der Handgriff (4, 6, 8) eine Unterseite (4a) aufweist, die geeignet ist, in Richtung des Zylinders (1) angeordnet zu werden, und eine Oberseite (4b) gegenüber der Unterseite (4a) aufweist; wobei der mindestens eine Handgriff (4, 6, 8) ein vertieftes Gehäuse aufweist, das zur Aufnahme des Kopfes (30) der Stange geeignet ist;
**dadurch gekennzeichnet,**
**dass** das vertiefte Gehäuse (40, 80) eine Öffnung (400) aufweist, die an einem Umfangsrand des Griffs (4; 6; 8) derart ausgebildet ist, dass der Griff (4; 6; 8) damit gekoppelt und entkoppelt werden kann, wobei sich der Kopf (30) der Stange (3) in einer radialen Richtung relativ zur Achse (Y) der Stange (3) verläuft.

2. Injektionsvorrichtung (100) nach Anspruch 1, wobei der mindestens eine Handgriff (4, 6, 8) zwei seitliche Elemente (47a, 47b) umfasst, die in entgegengesetzte Richtungen aus dem versenkten Gehäuse (40, 80) hervorstehen.

3. Injektionsvorrichtung (100) nach einem der vorhergehenden Ansprüche, wobei der Kopf (30) der Stange eine im Wesentlichen halbelliptische Form aufweist, wie die Spitze eines Daumens; wobei der Kopf (30) umfasst:
- eine gekrümmte hintere Kante (31) in Form eines Kreisbogens mit einem Mittelpunkt (O), der entsprechend der Achse der Stange (3) angeordnet ist,
- zwei gerade seitliche Kanten (32), und
- eine vordere Kante (33), die im Wesentlichen gerade ist oder die Form des Mittelteils einer Ellipse hat.

4. Injektionsvorrichtung (100) nach Anspruch 3, wobei das vertiefte Gehäuse (40, 80) zwei gerade seitliche Kanten (42) aufweist, die mit einer gekrümmten hinteren Kante (41) verbunden sind, wobei die seitlichen Kanten (43) einen konkaven C-förmigen Abschnitt haben und die Tiefe des vertieften Gehäuses (40) gleich der Dicke des Kopfes (30) ist.

5. Injektionsvorrichtung (100) nach einem der vorhergehenden Ansprüche, wobei das vertiefte Gehäuse (40, 80) auf der oberen Seite (4b) des mindestens einen Handgriffs (4, 6) ausgebildet ist; wobei der mindestens eine Handgriff (4, 6) einen Schlitz (44) umfasst, der mittig im vertieften Gehäuse vorgesehen ist; wobei der Schlitz (44) vorne und unten offen ist und in einem Loch (45) zur Aufnahme der Stange (30) endet.

6. Injektionsvorrichtung (100) nach Anspruch 5, wobei der mindestens eine Handgriff (4; 6) Haltezähne (49) aufweist, die in den Schlitz (44) hineinragen und elastisch verformt werden, um die Stange (3) festzuhalten.

7. Injektionsvorrichtung (100) nach Anspruch 5 oder 6, wobei der mindestens eine Handgriff (4; 6) einen Flansch (46) in Form eines offenen Rings aufweist, der in der unteren Position um das Loch (45) herum vorsteht.

8. Injektionsvorrichtung (100) nach einem der Ansprüche 5 bis 7, wobei der mindestens eine Handgriff einen ersten Handgriff (4) mit elliptischer Form umfasst, wobei die seitlichen Elemente (47a, 47b) mit abgerundeten Kanten (48a, 48b) versehen sind.

9. Injektionsvorrichtung (100) nach einem der Ansprüche 5 bis 7, wobei der mindestens eine Handgriff einen zweiten Handgriff (6) mit rechteckiger oder im Wesentlichen elliptischer Form umfasst, wobei die seitlichen Elemente (47a, 47b) mit geraden Kanten (48a, 48b) versehen sind.

10. Injektionsvorrichtung (100) nach einem der Ansprüche 1 bis 4, wobei das vertiefte Gehäuse (80) auf der unteren Seite (4a) des Handgriffs (8) derart ausgebildet ist, dass der Handgriff (8) vollständig den Kopf (30) nach oben ab deckt.

11. Injektionsvorrichtung (100) nach Anspruch 10, wobei der Handgriff (8) gegenüberliegende Kerben (89) aufweist, die an den seitlichen Kanten (42) des vertieften Gehäuses (80) angebracht sind; wobei der Kopf (30) Zähne (39) aufweist, die von den geraden seitlichen Kanten (32) nach außen vorstehen und in die Kerben (89) des Handgriffs (8) eingreifen.

12. Injektionsvorrichtung (100) nach einem der Ansprüche 10 bis 11, wobei der mindestens eine Handgriff einen dritten Handgriff (8) mit elliptischer Form umfasst, wobei die seitlichen Elemente (47a, 47b) mit abgerundeten Kanten (48a, 48b) versehen sind.

13. Injektionsvorrichtung (100) nach einem der vorhergehenden Ansprüche, wobei der Flansch (5) umfasst:
- ein zentral angeordnetes Loch (50), das von der Stange (3) durchdrungen werden kann;
- zwei seitliche Elemente (51a, 51b), die in entgegengesetzte Richtungen aus dem Loch (50) herausragen,
- einen Sitz (52), der mit dem Loch (50) in Verbindung steht und nach vorne offen ist, um einen Kragen (13) des Zylinders (1) aufzunehmen und festzuhalten,
- einen U-förmigen Schlitz (53), der in einer unteren Wand des Flansches angebracht ist, um mit dem Sitz (52) zum Einsetzen und Durchführen des Zylinders (1) in Verbindung zu stehen,
- Haltezähne (59), die gegenläufig in den Schlitz (53) hineinragen, um den Zylinder (1) festzuhalten, und
- einen Kragen (53) in Form eines geschlossenen Rings, der in der oberen Position des Flansches um das Loch (50) herum vorsteht, um die Gleitbewegung der Stange (3) zu führen.

14. Injektionsvorrichtung (100) nach einem der vorhergehenden Ansprüche, wobei der Zylinder (10) aus Glas besteht.

## Revendications

1. Dispositif d'injection (100) comprenant:
- un cylindre (1) avec une chambre interne (10),
- une tige (3) apte à coulisser à l'intérieur de la chambre (10) du cylindre; ladite tige présentant une extrémité proximale (3a) avec une tête (30) en forme de plaque orthogonale à un axe (Y) de la tige;
- une bride (5) apte à être fixée de manière amovible au cylindre (1),
- au moins une poignée (4; 6), de type interchangeable, apte à être fixée de manière amovible à la tige (3), en mode d'accouplement par emboîtement; ladite poignée (4, 6, 8) comprend un côté inférieur (4a) apte à être tourné vers le cylindre (1) et un côté supérieur (4b) opposé audit côté inférieur (4a); ladite au moins une poignée (4, 6, 8) comprend un boîtier encastré apte à recevoir ladite tête (30) de la tige;
**caractérisé en ce que**
ledit boîtier encastré (40, 80) présente une ouverture (400) qui est obtenue sur un bord périphérique de ladite poignée (4; 6; 8) de telle manière que ladite poignée (4; 6; 8) soit couplée et découplée avec ladite tête (30) de la tige (3) dans une direction radiale par rapport à l'axe (Y) de la tige (3) .

2. Dispositif d'injection (100) selon la revendication 1, dans lequel ladite au moins une poignée (4, 6, 8) comprend deux éléments latéraux (47a, 47b) qui font saillie dans des directions opposées dudit boîtier encastré (40, 80).

3. Dispositif d'injection (100) selon l'une quelconque des revendications précédentes, dans lequel ladite tête (30) de la tige présente une forme sensiblement semi-elliptique, comme le bout d'un pouce; ladite tête (30) comprenant:
- un bord arrière incurvé (31) en forme d'arc de cercle avec un centre (O) disposé en correspondance avec l'axe de la tige (3),
- deux bords latéraux droits (32), et
- un bord avant (33) qui est sensiblement droit ou en forme de partie centrale d'une ellipse.

4. Dispositif d'injection (100) selon la revendication 3, dans lequel ledit logement encastré (40, 80) comprend deux bords latéraux droits (42) qui sont joints à un bord arrière incurvé (41), dans lequel lesdits bords latéraux (43) ont une section en forme de C concave, et la profondeur dudit logement encastré (40) est égale à l'épaisseur de la tête (30).

5. Dispositif d'injection (100) selon l'une quelconque des revendications précédentes, dans lequel ledit boîtier encastré (40, 80) est obtenu sur le côté supérieur (4b) de ladite au moins une poignée (4, 6); ladite au moins une poignée (4, 6) comprenant une fente (44) ménagée en position centrale dans ledit boîtier encastré; ladite fente (44) étant ouverte à l'avant et dans le fond et se terminant par un trou (45) pour recevoir ladite tige (30).

6. Dispositif d'injection (100) selon la revendication 5, dans lequel ladite au moins une poignée (4; 6) comprend des dents de retenue (49) qui font saillie à l'intérieur de ladite fente (44) et sont déformées élastiquement pour retenir ladite tige (3).

7. Dispositif d'injection (100) selon la revendication 5 ou 6, dans lequel ladite au moins une poignée (4; 6) comprend une collerette (46) en forme d'anneau ouvert qui fait saillie en position inférieure autour dudit trou (45).

8. Dispositif d'injection (100) selon l'une quelconque des revendications 5 à 7, dans lequel ladite au moins une poignée comprend une première poignée (4) de forme elliptique, lesdits éléments latéraux (47a, 47b) étant pourvus de bords arrondis (48a, 48b).

9. Dispositif d'injection (100) selon l'une quelconque des revendications 5 à 7, dans lequel ladite au moins une poignée comprend une deuxième poignée (6) de forme rectangulaire ou sensiblement elliptique, lesdits éléments latéraux (47a, 47b) étant pourvus de bords droits (48a, 48b).

10. Dispositif d'injection (100) selon l'une quelconque des revendications 1 à 4, dans lequel ledit boîtier encastré (80) est obtenu sur le côté inférieur (4a) de la poignée (8) de telle manière que ladite poignée (8) recouvre complètement la tête (30) sur le dessus.

11. Dispositif d'injection (100) selon la revendication 10, dans lequel ladite poignée (8) comprend des encoches opposées (89) obtenues sur les bords latéraux (42) du boîtier encastré (80); ladite tête (30) comprenant des dents (39) qui font saillie extérieurement à partir des bords latéraux droits (32) et sont engagées dans les encoches (89) de la poignée (8) .

12. Dispositif d'injection (100) selon l'une quelconque des revendications 10 à 11, dans lequel ladite au moins une poignée comprend une troisième poignée (8) de forme elliptique, lesdits éléments latéraux (47a, 47b) étant pourvus de bords arrondis (48a, 48b).

13. Dispositif d'injection (100) selon l'une quelconque des revendications précédentes, dans lequel ladite bride (5) comprend:
- un trou (50) disposé en position centrale, apte à être pénétré par la tige (3);
- deux éléments latéraux (51a, 51b) qui font saillie dans des directions opposées à partir du trou (50),
- un siège (52) en communication avec le trou (50) et ouvert vers l'avant pour recevoir et retenir une collerette (13) du cylindre (1),
- une fente en U (53) obtenue dans une paroi inférieure de la bride, de manière à communiquer avec le siège (52) pour l'insertion et le passage du cylindre (1),
- des dents de retenue (59) qui font saillie dans la fente (53) dans des directions opposées pour retenir le cylindre (1), et
- une collerette (53) en forme d'anneau fermé qui fait saillie en position supérieure de la bride autour du trou (50) pour guider le mouvement de coulissement de la tige (3).

14. Dispositif d'injection (100) selon l'une quelconque des revendications précédentes, dans lequel ledit cylindre (10) est fait en verre.
